# NEW EUROPEAN PATENT SPECIFICATION

(11) **EP 1 523 339 B2**
(45) Date of publication and mention of the opposition decision: **14.12.2011**
(45) Mention of the grant of the patent: 27.12.2006
(21) Application number: 03740877.0
(22) Date of filing: 03.07.2003
(51) Int. Cl.: A61L 9/04, A61L 9/05, A23L 1/00, A23L 1/22

(54) **COMPOSITION FOR CONTROLLED RELEASE OF PERFUMES AND FLAVOURS**
ZUSAMMENSETZUNG FÜR DIE KONTROLLIERTE FREISETZUNG VON PARFÜMEN UND AROMEN
COMPOSITION PERMETTANT LA LIBERATION CONTROLEE DE PARFUMS ET DE FLAVEURS

(30) Priority: 11.07.2002 WO PCT/IB02/02860
(43) Date of publication of application: 20.04.2005
(73) Proprietor: Firmenich SA, 1211 Genève 8 (CH)
(72) Inventor: HOLZNER, Günter, CH-1212 GRAND-LANCY (CH); STRUILLOU, Arnaud, F-74160 ARCHAMPS (FR); DORMAL, Marc, CH-1205 GENEVE (CH); BOUQUERAND, Pierre-Etienne, F-74930 PERS-JUSSY (FR); BENCZEDI, Daniel, CH-1228 PLAN-LES-OUATES (CH)
(74) Representative: Salvaterra-Garcia, Maria de Lurdes
(86) International application number: PCT/IB2003/002630
(87) International publication number: WO 2004/006967

(56) References cited:
- EP-A- 0 492 981
- EP-A- 1 048 687
- WO-A-99/49850
- WO-A1-03/043728
- FR-A1- 2 791 906
- US-A- 5 723 420

## Description

### Technical field

The invention concerns an extruded composition capable of releasing a perfume or flavour when in contact with water.

The extruded composition according to the invention comprises:
a) a water-soluble polymeric element able to be processed by extrusion, and
b) a perfuming or flavouring element containing a perfuming or flavouring ingredient or composition, a hydrophilic hollow silica having a mean diameter comprised between 150 µm and 350 µm, and optionally a surfactant.

The composition can also comprise a dye as optional component.

The invention also concerns the use of said composition as a means to protect a perfume or flavour from an aggressive agent or medium, such as a bleach in the case of a perfume. Moreover, the invention also concerns consumer articles or food containing, or associated with, said composition, in particular powder detergents or soap bars for the perfumery, and chewing gums or candies for flavours.

### Prior art

Compositions which are able to release perfumes have become very common features nowadays in the perfume industry and are very frequently associated with detergents. However, said compositions present the inconvenient of needing large amounts of materials to deliver only a limited amount of perfume. One can cite the case of detergents wherein the amount of perfume, which is carried by and delivered from said compositions to the surface that is washed, is often small when compared with the total weight of the compositions.

In patent application EP 728804 there are disclosed compositions which tend to partially solve the problem mentioned hereinabove. The compositions described in said document are capable of containing up to 20% by weight of a perfume and also have the advantage of being of simple formulation, contrary to many other compositions disclosed in other prior art documents. More precisely, said compositions comprise a water-soluble polyvinyl polymer, i.e. a hydrophilic polymer, which, contains up to 20% by weight of a fragrance. In order to incorporate into the hydrophilic polymer such an amount of perfume, which is in general a hydrophobic liquid, the latter is previously absorbed into a hydrophobic silica.

However, the compositions disclosed in EP 728804, despite their advantages, still do not represent an optimal solution to the mentioned problem as they deliver only up to a fifth of their weight of perfume in the best cases, i.e. 80% of their weight is lost in carrier materials which will be dispersed into the environment.

Therefore, there is still a need, especially for the detergent industry, for a composition which is capable of carrying and delivering high amounts of a perfuming ingredient in order to limit the total mass of the composition, and consequently the waste into the environment, while maintaining high performance of fragrance delivery.

A similar problematic is known in the flavour industry where there is a constant need for improving the loadings and delivery of flavour ingredients or compositions from the same type of compositions.

### Description of the invention

We have now surprisingly discovered that, by using a hydrophilic hollow silica to absorb a hydrophobic perfume or flavour, rather than a hydrophobic silica as in the prior art, it is possible to obtain a water-soluble composition of simple formulation and capable of carrying high amounts of perfume or flavour, thus limiting the amount of carrier or wastes which will be dispersed in the environment.

The extruded composition according to the invention comprises:
a) a water-soluble polymeric element able to be processed by extrusion, and
b) a perfuming or flavouring element containing:
   i) a perfuming or flavouring ingredient or composition,
   ii) a hydrophilic hollow silica having a mean diameter comprised between 150 µm and 350 µm, and
   iii) optionally a surfactant.

The composition can also comprise a dye as optional component.

The water-soluble polymeric element is not only intended to carry the other ingredients of the invention composition, but also to protect the integrity of the perfuming or flavouring element during the manufacture and the storage of the composition or consumer article according to the invention as described further below.

An appropriate polymeric element for the invention must be able to be processed by extrusion, in order to allow the inclusion of the perfuming or flavouring ingredients into the polymer mass. Additionally, said polymeric element must be water-soluble, so that once the composition of the invention is in contact with a sufficient amount of water, said polymeric element will dissolve, thus liberating the perfuming or flavouring element and consequently releasing the perfume or flavour into the surrounding environment, for instance on a washed surface or yet in the mouth during the consumption of food. By "water-soluble polymeric element" it is meant here a polymeric element which is almost entirely, i.e. more than 90% of its total weight, dissolved in water at 90°C and at a 10% weight/weight concentration.

Ideally, the polymeric element consists of a water-soluble polymer.

As an example of a water-soluble polymer according to the invention, one can cite any grade of water-soluble polyvinyl alcohol or water-soluble partially hydrolysed polyvinyl acetate; said polymers having also the advantage of being biodegradable. Both polyvinyl alcohol and partially hydrolysed polyvinyl acetate are obtained by hydrolysis of polyvinyl acetate. Preferably, the water-soluble polymer of the invention has a percentage of hydrolysed acetate groups comprised between 80% and 99%, most preferably between 85% and 99%.

The polyvinyl alcohol and partially hydrolysed polyvinyl acetate are known to modify the viscosity of water when dissolved. Preferably the water-soluble polymer of the invention is such that, when dissolved at a concentration of 4% weight/weight in water at 20°C it provides solutions having a viscosity lower than 40 mPa·s, more preferably lower than 20 mPa·s.

Examples of commercially available water-soluble polymers, which are useful for the invention, are known under the tradename Mowiol^{®} 10/98, Mowiol^{®} 4/88, Mowiol^{®} 8/88 (origin: Clariant, Germany) or yet Soltec^{®} T10 (origin: Soltec Développement SA, France) or Solublon^{®} (origin Aicello, Japan).

More frequently, the water-soluble polymeric element will also contain a plasticiser in order to facilitate the extrusion process of the composition. One can cite, as non-limiting examples of plasticisers, water, glycerine, ethylene glycol, propylene glycol, dipropylene glycol or diethylene glycol. Preferably the plasticiser will be water or glycerine.

The amount of plasticiser present in the polymeric element may vary between 0% to 20%, preferably between 1% to 10%, the percentage being relative to the weight of the polymeric element.

The compositions of the invention may contain the polymeric element in an amount comprised between 40 % and 70 %, the percentages being relative to the total weight of the composition. In a preferred embodiment of the invention, the polymeric element is present in an amount comprised between 45 % and 55 %.

The perfuming or flavouring element contains two different ingredients, each of them having a precise function. The perfuming or flavouring element in the final extruded composition is essentially included or contained into the water-soluble polymeric element.

The first ingredient of the perfuming or flavouring element is a perfuming or flavouring ingredient or composition. It must be properly understood that a perfuming or flavouring composition, within the framework of the invention, designates a composition essentially comprising ingredients currently used in perfumery or flavour industry and able to impart an odour, or to improve, enhance or modify the odour or taste properties of the composition to which they are added. Usually, these ingredients will form a more or less complex mixture of volatile ingredients of natural or synthetic origin. The nature of these ingredients can be found in specialised books, e.g. in S. Arctander (Perfume and Flavor Chemicals, Montclair N.J., USA 1969), or in Fenaroli's Handbook of Flavour Ingredients, CRC Press or yet in Synthetic Food Adjuncts by M.B. Jacobs, van Nostrand Co., Inc. or similar textbooks of reference, and a more detailed description thereof is not warranted here. Additionally, useful perfuming or flavouring ingredients will be in a liquid form.

The amount of perfuming or flavouring ingredient present in the perfuming or flavouring composition may vary between 50% and 100%, preferably between 90% and 100%, the percentage being relative to the weight of the perfuming or flavouring composition.

The perfuming or flavouring composition may further comprise one or more additional ingredients such as antioxidant agents, antibacterial or bacteriostatic agents, insect repellents or cosmetic or skin care ingredients.

As non-limiting examples of antioxidants one can mention Tocopherol, BHT (Butylhydroxytoluene) DLTDP (Dilaurylthiodipropionate). As cosmetic skin caring ingredients one can mention emollients, lanolin, esters of fatty acids with different mono, di and trivalent alcohols, vaseline oil, squalane, olive oil and others, moisturisers, amino acids, glycerine, sorbitol, polyvinylpyrrolidone, plant extracts such as aloe, hamamelis, horse chestnut, ginko biloba and many others and vitamins such as Vitamin C and its ester, Vitamin E acetate, Vitamin F (unsaturated fatty acids) and others. Regarding the antibacterials or bacteriostatics effect of the perfuming ingredient, it is useful to mention that the perfume itself may possess such effects.

The amount of additional ingredients present in the perfuming or flavouring composition may vary between 0% and 50%, preferably between 0% and 10%, the percentage being relative to the weight of the perfuming or flavouring composition.

According to the invention, the perfuming or flavouring ingredient or composition may be incorporated in the perfuming or flavouring element in an amount comprised between 20 % and 70 %, the percentages being relative to the total weight of the perfuming or flavouring element. Preferably, the perfuming or flavouring ingredient or composition is present in amounts comprised between 30 % and 70 %, even more preferably between 50 % and 70 %, the percentages being relative to the total weight of the perfuming or flavouring element.

The second ingredient of the perfuming or flavouring element is a particular type of silica, namely a hydrophilic hollow silica. The hollow silica is intended to contain or absorb the perfuming or flavouring ingredient or composition.

Useful hydrophilic hollow silica have an essentially spherical morphology and have a mean diameter comprised between 150 µm and 350 µm, preferably between 200 µm and 300 µm. The morphology of said hydrophilic hollow silica is thus similar to that of a micropearl.

The size and the hydrophilicity of the hollow silica are important aspects of the latter. Indeed we have surprisingly found that by using such large hollow particles, instead of an amorphous silica or smaller silica particles, it was possible to extrude the composition of the invention without observing any appreciable leak of perfume or flavour from the silica, even when high amounts of perfume or flavour were incorporated within. The hydrophilicity of the hollow silica allows a better release into the surrounding environment of the perfume or flavour which is, in general, a hydrophobic liquid. The specific properties of the hollow silica used in the invention permit therefore to obtain an extruded composition capable of incorporating high amounts of perfume or flavour and also to release, or deliver into the surrounding environment, said perfume or flavour in an efficient manner.

In addition to the above-mentioned properties, preferably the silica also has a high oil absorption capacity, i.e. it is capable of absorbing oil in more than two times its own weight. The oil absorption capacity may be measured using dioctylphthalate as reference compound, according to a method described in WO 99/49850, and is measured in g/100g of silica or in ml/100 g of silica. Thus; the silica of the invention has, preferably, an oil absorption capacity comprised between 230 ml/100 g and 350 ml/100 g, or between 230 g/100 g and 350 g/100 g.

Said hydrophilic hollow silica is obtainable by spray drying of precipitated silica as described in WO 99/49850, the contents of this preparation there-described is incorporated herein by reference.

Examples of commercially available hydrophilic hollow silica useful to the invention are known under the tradenames Sipernat^{®} 2200 (origin: Degussa, Germany) or Tixosil^{®} 38X or 68 (origin: Rhodia, France).

The hydrophilic hollow silica may be incorporated in the perfuming or flavouring element according to the invention in an amount comprised between 30 % and 70 %, the percentages being relative to the total weight of the perfuming or flavouring element. Preferably, the hydrophilic hollow silica is present in amounts comprised between 30 % and 50 %, even more preferably between 30 % and 40 %, the percentages being relative to the total weight of the perfuming or flavouring element.

The presence of a surfactant in the perfuming or flavouring element is optional, but preferred, and is intended to help the absorption of the hydrophobic perfume or flavour by the hydrophilic hollow silica. As non-limiting examples, the surfactant may be selected from the group consisting of polyalkyleneglycol ethers of a C₁-C₁₀ alkanol, polyalkyleneglycol ethers of synthetic C₉₋₂₀ fatty alcohols and mono laurate esters of sorbitol condensed with polyethylene oxide. Preferably, the surfactant is selected from the group consisting of polysorbates, PPG/Buteth derivatives, and Pareth derivatives.

Examples of commercially available surfactants, which are useful to the invention, are known under the tradename Tergitol^{®} 15-S-9 (C₁₁₋₁₅ Pareth-9) (origin: Union Carbide, USA), Tergitol^{®} XD (PPG-24-Buteth-27) (origin: Union Carbide, USA) or Tween^{®} 20 (origin: ICI, UK).

The surfactant may be incorporated into the perfuming or flavouring element according to the invention in an amount comprised between 0% and 10%, the percentages being relative to the total weight of the perfuming or flavouring element Preferably, the surfactant is present in amounts comprised between 3% and 8%, the percentages being relative to the total weight of the perfuming or flavouring element.

The compositions of the invention may contain the perfuming or flavouring element in an amount comprised between 30% and 70%, the percentages being relative to the total weight of the composition. In a preferred embodiment of the invention, the perfuming or flavouring element is present in an amount comprised between 45% and 55% by weight, relative to the total weight of the composition.

It is thus possible to obtain compositions according to the invention having a very high load of perfume or flavour, i.e. up to approximately 40%. Such levels of perfume or flavour imply that, to deliver the same amount of perfume or flavour, a composition according to the invention needs half the weight of carrier when compared with a composition disclosed in EP 728804.

Compositions according to the invention having a perfume or flavour content comprised between 25% and 35% of the composition's total weight are preferred.

The compositions of the invention may further comprise, as optional component, a dye, preferably a hydrophilic one, such as a naphthalene or trityl derivative. Non-limiting examples of suitable dyes are Patent Blue V [trityl derivative: N-[4-[[4-dietheylamino)phenyl](5-hydroxy-2,4-disulfophenyl)methylene]2,5-cyclohexadien-1-ylidene]-N-ethylethanaminium, hydroxide, inner Salt or calcium Salt (2:1)], Food Green 3 [trityl derivative: benzenemethanaminium, N-ethyl-N-[4-[[4-[ethyl[(3-sulfophenyl)methyl]amino]phenyl](4-hydroxy-2-sulfophenyl)methylene]-2,5-cyclohexadien-1-ylidene]-3-sulfo-, hydroxide, inner Salt or disodium Salt], Fuchsine [trityl derivative: 4-[(4-aminophenyl)(4-imino-2,5-cyclohexadien-1-ylidene)methyl]-2-methylbenzenamine monohydrochloride] or Orange 6 [naphthalene derivative: (disodium 7-hydroxy-8-(phenylazo)-1,3-naphthalenedisulfonate)].

The dye component may be incorporated into the composition of the invention in an amount comprised between 0% and 1%, the percentages being relative to the total weight of the composition. Preferably, the dye component is present in amounts comprised between 0.005% and 0.5%.

The composition of the invention may be obtained by extruding a mixture of its constituents. The extrusion may be performed according to a "master batch" method, i.e. addition into the extruder of a mixture of all the elements, or alternatively according to a "side feeding" method, wherein the polymeric element is introduced into the extruder and, downstream, combined with the perfuming or flavouring element.

As extruder devices there may be used single or double screw extruders. Examples of such extruders are described in Schwaxz/Ebeling/Lupke/Schelter: Kunststoffverarbeitung, 2. Ed., Vogel Verlag, 1983.

The extruder is equipped with a temperature regulation mechanism so that it is possible to maintain the temperature of the mixture in a range comprised between 90°C and 270°C, to form a molten mass. As the molten mass exits the extruder, it is cooled, using standard methods which do not need water, and it can be chopped into a multitude of granules by using an appropriate apparatus, to obtain a composition of the invention in a granular form. The size and diameter of the granules will depend on the opening of the exit nozzle and the stroke rate of the cutting apparatus; said size is preferably comprised between 0.1 mm and 10.0 mm; more preferably between 1 mm and 5.0 mm.

Different forms of the granules or flakes, optionally with an aesthetic function, eg. flowers or stars etc, can be obtained by combining the adequate extruder opening with cutting devices.

As anticipated above, the composition of the invention, preferably in the form of granules or flakes, may be contained in, or associated with, a consumer article such as in perfumery, a solid detergent in the form of tablets, a powder or a bar and in the case of flavours, a food.

Solid detergents, constitute another object of the invention and may be intended for textile or skin treatment, or also for cleaning dishes or varied surfaces, for industrial or household use.

Tablets of detergents may be obtained according to any current method of production of such tablets.

Powder detergents according to the invention may be obtained by admixing the composition of the invention, in a granular form, with said powder. Thus, it will be obtained a powder detergent containing, as perfume dispenser, the composition of the invention.

Detergent bars, such as soaps, according to the invention may be obtained by extruding the composition of the invention, in a granular form, together with the detergent mass. Detergent bars represent an unexpected application of the invention compositions. Indeed the extrusion of soaps required very high pressures which generally break the most of the capsule or microsponge systems known. The invention's extruded granules or flakes resist to the pressure and shearing action of standard bar soap manufacturing extruders.

According to their compositions, detergents may be considered as aggressive media for perfumes. Indeed strong bases or bleaches, which may be present in the detergent formulation, can chemically degrade the perfume with the undesired consequence of changing the perfuming effect that the latter can confer to a treated surface. Said degradation may occur upon storage and/or upon use of the detergent. Generally, the degradation observed upon storage is more important due to the longer time of contact between the perfume and the detergent.

In the case of flavours, the compositions of the invention may be added to sweet applications such as chewing gums in particular. Chewing gum compositions are obtained by conventional methods, well known by a skilled person in the art.

Now, we have surprisingly discovered that the invention compositions are also able to protect the perfume or flavour from chemical degradation. In the perfumery field, such degradation may be caused by some ingredients present in a detergent, such a protection being thus particularly effective during the storage of the invention's detergent. The protective action is believed to be due to the specific nature of the invention composition wherein the perfume is protected twice, by the polymer and by the hollow silica, from an accidental contact with aggressive ingredients of the medium, thus avoiding, minimizing or delaying the mechanisms responsible for the degradation. It is also possible that some interactions between the polymer or the silica and the perfume somehow stabilise the latter, making it less sensitive to the action of aggressive ingredients such as oxidants. Similarly in the case of flavouring ingredients, losses of volatile components from food products may produce undesirable variations in the taste and aroma of the products as perceived by the consumer. On the other hand, losses of volatile components might occur through the conversion of certain flavour materials into unwanted less desirable or tasteless chemicals by their interaction with reagents present in the environment. Oxygen is an example of this type of reagent as it promotes the conversion of several labile flavour materials of current and critical utilisation in the industry. Therefore, it is always useful to be able to efficiently protect flavouring ingredients from degradation, during storage and before their consumption.

Consequently, another object of the invention is the use of an extruded composition according to the invention as a means to protect a perfume or flavour from an aggressive medium or agent, such as a bleach, oxygen or a strong base.

Another advantage of the invention resides in the fact that the consumer products containing an extruded composition according to the invention are able to impart a more intense fragrance or flavour, unlike the effect obtained with a consumer product perfumed or flavoured strictly with the fragrances or flavours as such.

Moreover, we have also discovered that in the case of perfumes, the above-mentioned protective action is also effective upon use of the detergent. Indeed, by carefully choosing the size of the granule, or the type of water-soluble polymer, it is possible to adjust the time needed by an extruded composition according to the invention to release the perfuming ingredient or composition, once said composition is in contact with water, such as during the use of the detergent. We have thus found that it is possible to obtain compositions according to the invention that may deliver the perfume to the treated surface in a period of time comprised between 0.5 and 30 minutes, or even 60 or 90 minutes after the contact with water.

This behaviour is very attractive since it allows to use a composition according to the invention either for a fast release or for a slow release of the perfume.

The slow release of the perfume is particularly attractive as it allows the delivery of the perfume on the treated surface once the most aggressive ingredients of the detergent have been washed out or consumed, thus minimising the chance of the perfume degradation upon use of the detergent.

Therefore, due to the particular behaviour of the invention composition which allows to minimise the chance of the perfume degradation upon storage and use, to choose the time of release of the perfume, and eventually also the kinetic of said release, yet another object of the invention is a process for the perfuming of a surface or a process for intensifying, prolonging or deferring the diffusion effect of the characteristic, fragrance of a perfume on a surface, characterised in that said surface is treated in the presence of an extruded composition according to the invention.

In the case of flavours, the compositions of the invention also proved to be very advantageous as they allow to provide a particularly intense flavouring effect compared with the case wherein the flavour ingredient or composition is used in a free form.

The invention will now be described in further detail by way of the following examples wherein temperatures are given in degrees Celsius, and the abbreviations have the usual meaning in the art.

### Example 1

### Manufacture of extruded compositions according to the invention

### The perfuming element

A perfuming element was obtained by admixing, in the given sequence, in a beaker the following ingredients to allow a total absorption of the fragrance into hollow silica:

| | |
|---|---|
| Fragrance* | 325 g |
| Tween^{®} 20 ¹⁾ | 25 g |
| Sipernat^{®} 2200 ²⁾ | 150 g |
| | 500 g |

| | |
|---|---|
| 1) polyoxyethylene(20)sorbitan monolaurate ; origin : ICI, UK 2) hollow silica, origin : Degussa, Germany * Fragrance composition | |

| Ingredient | Part by weight % |
|---|---|
| 4-Dimethyl-3-cyclohexene-1-carbaldehyde | 10 |
| Benzyl Salicylate | 10 |
| Lilial^{®} (origin : Givaudan) 3-(3-Isopropyl-1-phenyl)butanal | 10 |
| (+-)-3-(4-isopropylphenyl)-2-methylpropanal | 10 |
| 2-Methyl undecanal | 10 |
| Eugenol | 10 |
| Benzyl acetate | 10 |
| Hexyl salicylate | 10 |
| Phenylethyl acetate | 10 |
| | 100 |

### The extruded compositions

a) 250 g of above perfuming element were mixed with 250 g of a polyvinyl alcohol (Soltec^{®} T10, Soltec SA, France) and with 1 g of a dye (Vibracolor blue PBL 15/3 L, Ciba AG, Switzerland) to obtain blue coloured granules.
   Finally the whole mixture was extruded in a 2-screw Brabender (Germany) laboratory
   extruder with the following temperatures :
   Extruder zone 1 = 200°; zone 2 = 220°, zone 3 = 180°. With an extruder outlet of 5 mm, a string of the same diameter was extruded and immediately cut to obtain granules of the same diameter containing 32.5% of fragrance.
b) 200 g of the above perfuming element were mixed with 400 g of polyvinyl alcohol (Soltec^{®} T10, Soltec SA, France) and with 0.2 g of a dye (Vibracolor yellow PYE 1-L, Ciba AG Switzerland) and 0.8 g of another dye (Vibracolor green PGR 7-L, Ciba AG Switzerland) to obtain a green colour.
   The extrusion was performed with the same conditions as part a) above but with an extruder outlet allowing the formation of a flat film with 0.3 - 0.4 mm thickness containing 20% fragrance. The film was immediately cut in small flakes of 4 - 5 mm size.

### Example 2

### Stability of PVOH extruded fragrance in a detergent powder

Various invention compositions, obtained according to example 1b), were admixed with a standard European detergent powder with percarbonate-TAED bleaching system. After two weeks storage at 37°C and 70% of relative humidity, the invention compositions were separated from the detergent, dissolved in water and diluted with acetonitrile and 2-heptanone. 1 g of the solution thus obtained was dried over 5 g of NaSO₄ and the residual liquid phase was analysed by a standard GC-MS analytical method to determine the residual quantity of the various fragrant aldehydes. The results were compared with those obtained with a reference detergent, stored for two weeks at 3°, on which the perfume was directly sprayed-on. The results are summarised in Table 1.

**Table 1 : Percentage of the total aldehyde recovered after storage**

| Aldehyde | Reference (sprayed-on) | Granules with 20% perfume loading | Granules with 25% perfume loading | Granules with 32.5% perfume loading |
|---|---|---|---|---|
| 2-Methyl-undecanal | 0% | 28% | 31% | 41% |
| 3-(3-Isopropyl-1-phenyl)butanal | 6% | 44% | 44% | 50% |
| Cyclosal | 5% | 28% | 28% | 31 % |
| Lilial® | 6% | 35% | 34% | 37% |
| Time needed for full perfume release | <10 minutes | ∼30 minutes | ~30 minutes | >30 minutes |

As it can be noticed, when compared to the reference, the granules according to the invention are always able to deliver higher quantities of fragrant aldehydes by decreasing considerably the degradation of the latter, and are also able to delay the release of said aldehydes in water, thus decreasing also the probability of contact between said aldehydes and the bleaches.

### Example 3

### Manufacture of extruded compositions according to the invention

A perfuming element was obtained by admixing, in the given sequence, in a beaker, the following ingredients to allow a total absorption of the fragrance into hollow silica :

| Ingredients | grams |
|---|---|
| Fragrance¹⁾ | 360 |
| Sipernat^{®} 2200²⁾ | 200 |

| | |
|---|---|
| 1) fleur de menthe 68528 E : origin : Firmenich SA, Geneva, Switzerland 2) hollow silica ; origin : Degussa, Germany | |

The perfuming element was mixed with 337 g of polyvinyl alcohol (V03/180, origin : Erkol SA, Spain), 60 g of water and with 3 g of dye (1 g of vibracolor vert PGR7L et 2g of vibracolor jaune PYE13L ; origin: Ciba AG, Switzerland) to obtain green coloured granules.

Finally, the whole mixture was extruded in a 2-screw PRISM-EuroLab extruder with the following process parameters : temperature 108°; pressure 10-15x10⁵ Pa; screw rpm 180; die hole 1.5 mm.

The capsules obtained were characterised by 26 to 28% by weight of perfume load relative to the total weight of the capsules.

### Example 4

### Manufacture of extruded compositions according to the invention

A perfuming element was obtained by admixing, in the given sequence, in a beaker, the following ingredients to allow a total absorption of the fragrance into hollow silica:

| Ingredients | grams |
|---|---|
| Fragrance¹⁾ | 187 |
| Sipernat^{®} 2200 ²⁾ | 93 |

1) fragrance composition :

| Ingredients | Parts by weight |
|---|---|
| Verdox^{®} (origin : IFF, USA) | 42.6 |
| Allyl heptanoate | 25.5 |
| Hexyl salycilate | 14.9 |
| Phenoxy isobutyrate | 17.0 |
| Total | 100.0 |

2) hollow silica: origin: Degussa, Germany

The perfuming element was mixed with 654 g of polyvinyl alcohol (V03/180, origin: Erkol SA, Spain) and 66 g of water.
Finally, the whole mixture was extruded in a 2-screw PRISM-EuroLab extruder with the following process parameters : temperature 108°; pressure 10-15x10⁵ Pa ; screw rpm 180; die hole 1.5 mm.
The obtained granules were loaded with 15% of fragrance.

### Example 5

### A soap bar containing a composition according to the invention

The granules and flakes from example 3 were incorporated at 1% in a classical translucent soap base type 1984 from Uniqema (The Netherlands).
The soap extrusion was performed at a temperature comprised between 45° and 50°C on a Beck laboratory extruder type BV45 made by Ehrismann SA, Switzerland. Soap bars of 90 g were pressed on a RMT laboratory soap press (RMT Ltd. Great Britain). The granules and flakes were not broken and gave a pleasant visual effect to the soap bar

### Example 6

### Manufacture of extruded compositions according to the invention

A flavouring element was obtained by admixing, in the given sequence, in a beaker, the following ingredients to allow a total absorption of the flavour into hollow silica:

| Ingredients | grams |
|---|---|
| Flavour ¹⁾ | 10 |
| Cooling agent ²⁾ | 40 |
| Sipernat^{®} 2200³⁾ | 93 |

| | |
|---|---|
| 1) 758810 01T; origin: Firmenich SA, Geneva, Switzerland 2) 927875 ; origin : Firmenich SA, Geneva, Switzerland 3) hollow silica; origin: Degussa, Germany | |

The flavouring element was admixed with 870 g of polyvinyl alcohol (V03/180, origin : Erkol SA, Spain) 60 g of water, 5 g of Citrem^{®} (origin: Danisco, Denmark) and 5 g of fractionated coconut oil (origin : Stearineric Dubois).
Finally, the whole mixture was extruded in a 2-screw PRISM-EuroLab extruder with the following process parameters : temperature 130°; pressure 10-15x10⁵ Pa ; screw rpm 180 ; die hole 1.5 mm.

### Example 7

### Application of flavoured capsules according to the invention in chewing gums

A chewing gum was formulated with the following ingredients :

| Ingredients | Parts by weight |
|---|---|
| Gum base ¹⁾ | 250 |
| Sorbitol powder | 523 |
| Sorbitol syrup | 150 |
| Glycerine | 65 |
| Aspartame^{®} | 2 |
| Acesulfam-K | 2 |
| Flavoured capsules²⁾ | 8 |
| Total | 1000 |

| | |
|---|---|
| 1) L.A. Dreyfus Nova 2) prepared according to Example 6 | |

The gum base was added to a classical gum mixer and mixed until homogeneous. Half of the sorbitol powder and half of the syrup were added and the whole mixture was mixed for 5 to 8 minutes. The glycerine was added and mixed one minute. The flavoured capsules were added and mixed for 4 to 5 minutes. The final mixture was removed from mixer, shape cut and wrap.

The chewing gum obtained proved to have an increased flavour impact without bitterness and longer lasting flavour duration compared with similar products with free flavours.

## Claims

1. An extruded composition comprising:
a) a water-soluble polymeric element able to be processed by extrusion, and
b) a perfuming or flavouring element containing:
i) a perfuming or flavouring ingredient or composition,
ii) a hydrophilic hollow silica having a mean diameter comprised between 150 µm and 350 µm, and
iii) optionally a surfactant.

2. An extruded composition according to claim 1, wherein the water-soluble polymeric element consists of a water-soluble polymer.

3. An extruded composition according to claim 2, wherein the water-soluble polymer is a water-soluble polyvinyl alcohol or a water-soluble partially hydrolysed polyvinyl acetate.

4. An extruded composition according to claim 2, wherein the polymeric element further comprises a plasticiser selected from the group consisting of water, glycerine, ethylene glycol, propylene glycol, dipropylene glycol or diethylene glycol.

5. An extruded composition according to claim 1, comprising a perfuming element containing a perfuming composition.

6. An extruded composition according to claim 5, wherein the perfuming composition comprises at least one perfuming ingredient and one or more additional ingredients selected from the group consisting of antioxidant agents, antibacterial or bacteriostatic agents, insect repellents or cosmetic or skin care ingredients.

7. An extruded composition according to claim 1, wherein the hydrophilic hollow silica has an oil absorption capacity comprised between 230 ml/100 g and 350 ml/100 g.

8. An extruded composition according to claim 1, wherein the surfactant is selected from the group consisting of polyalkyleneglycol ethers of a C₁-C₁₀ alkanol, polyalkyleneglycol ethers of synthetic C₉₋₂₀ fatty alcohols and mono laurate esters of sorbitol condensed with polyethylene oxide.

9. An extruded composition according to claim 1, wherein the perfume content is comprised between 25% and 35% of the composition's total weight.

10. An extruded composition according to claim 1, further comprising a dye.

11. An extruded composition according to any one of claims 1 to 10, in a granular form wherein the granules have a size preferably comprised between 0.1 mm and 10.0 mm.

12. Use of an extruded composition according to any one of claims 1 to 11, as a means intended to protect a perfume or flavour from an aggressive medium.

13. A process for the perfuming of a surface or a process for intensifying, prolonging or deferring the diffusion effect of the characteristic fragrance of a perfume on a surface, **characterised in that** said surface is treated in the presence of an extruded composition according to anyone of claims 1 to 11.

14. A consumer article or a food containing an extruded composition according to any one of claims 1 to 11.

15. A consumer article according to claim 14, in the form of tablets, a powder or bar detergent.

16. A food according to claim 14, in the form of a chewing gum.

## Patentansprüche

1. Extrudierte Zusammensetzung, aufweisend:
(a) einen wasserlöslichen polymeren Bestandteil, der fähig ist, durch Extrusion behandelt zu werden, und
(b) einen parfümierenden oder Aroma vermittelnden Bestandteil, enthaltend:
(i) einen parfümierenden oder Aroma vermittelnden Inhaltsstoff oder eine solche Zusammensetzung,
(ii) hydrophiles, hohles Siliziumdioxid mit einem mittleren Durchmesser von 150 µm bis 350 µm, und
(iii) wahlweise ein Tensid.

2. Extrudierte Zusammensetzung nach Anspruch 1, worin der wasserlösliche polymere Bestandteil aus einem wasserlöslichen Polymer besteht.

3. Extrudierte Zusammensetzung nach Anspruch 1, worin das wasserlösliche Polymer ein wasserlöslicher Polyvinylalkohol ist oder ein wasserlösliches, teilhydrolysiertes Polyvinylacetat.

4. Extrudierte Zusammensetzung nach Anspruch 2, worin der wasserlösliche polymere Bestandteil ferner einen Weichmacher aufweist, der ausgewählt ist aus der Gruppe, bestehend aus Wasser, Glycerin, Ethylenglykol, Propylenglykol, Dipropylenglykol oder Diethylenglykol.

5. Extrudierte Zusammensetzung nach Anspruch 1, aufweisend einen parfümierenden Bestandteil, der eine parfümierende Zusammensetzung enthält.

6. Extrudierte Zusammensetzung nach Anspruch 5, worin die parfümierende Zusammensetzung mindestens einen parfümierenden Inhaltsstoff aufweist und einen oder mehrere zusätzliche Inhaltsstoffe, die ausgewählt sind aus der Gruppe, bestehend aus Antioxidantien, antibakteriellen oder bakteriostatischen Mitteln, Insektenabwehrmitteln oder Inhaltsstoffen der Kosmetik und Hautpflege.

7. Extrudierte Zusammensetzung nach Anspruch 1, worin das hydrophile, hohle Siliziumdioxid ein Öl-Aufnahmevermögen zwischen 230 ml/100g und 350 ml/100g hat.

8. Extrudierte Zusammensetzung nach Anspruch 1, worin das Tensid ausgewählt ist aus der Gruppe, bestehend aus Polyalkylenglykolethern eines C₁-C₁₀-Alkanols, aus Polyalkylenglykolethern von synthetischen C₉-C₂₀-Fettalkoholen und Monolauratestern von Sorbit, kondensiert mit Polyethylenoxid.

9. Extrudierte Zusammensetzung nach Anspruch 1, worin der Parfümgehalt zwischen 25% und 35% des Gesamtgewichts der Zusammensetzung ausmacht.

10. Extrudierte Zusammensetzung nach Anspruch 1, ferner aufweisend einen Farbstoff.

11. Extrudierte Zusammensetzung nach einem der Ansprüche 1 bis 10 in granularer Form, wobei die Granalien vorzugsweise eine Teilchengröße zwischen 0,1 und 10,0 mm haben.

12. Verwendung einer extrudierten Zusammensetzung nach einem der Ansprüche 1 bis 11 als ein Mittel, das zum Schutz eines Parfüms oder Aromas gegen ein aggressives Medium vorgesehen ist.

13. Verfahren zum Parfümieren einer Oberfläche oder Verfahren zum Intensivieren, Verlängern oder Verzögern der Diffusionswirkung des charakteristischen Duftstoffes eines Parfüms auf einer Oberfläche, **dadurch gekennzeichnet, dass** die Oberfläche in Gegenwart einer extrudierten Zusammensetzung nach einem der Ansprüche 1 bis 11 behandelt wird.

14. Verbrauchsartikel oder Lebensmittel, enthaltend eine extrudierte Zusammensetzung nach einem der Ansprüche 1 bis 11.

15. Verbrauchsartikel nach Anspruch 14 in Form von Waschmitteltabletten, -pulver oder -riegel.

16. Lebensmittel nach Anspruch 14 in Form eines Kaugummis.

## Revendications

1. Composition extrudée comprenant:
(a) un élément polymère hydrosoluble capable d'être traité par extrusion, et
(b) un élément parfumant ou aromatisant contenant:
(i) un ingrédient ou une composition parfumants ou aromatisants,
(ii) une silice creuse hydrophile ayant un diamètre moyen compris entre 150µm et 350µm, et
(iii) optionnellement un agent tensioactif.

2. Composition extrudée selon la revendication 1, dans laquelle l'élément polymère hydrosoluble consiste en un polymère hydrosoluble.

3. Composition extrudée selon la revendication 2, dans laquelle le polymère hydrosoluble est un alcool polyvinylique hydrosoluble ou un polyvinyle acétate hydrosoluble partiellement hydrolysé.

4. Composition extrudée selon la revendication 2, dans laquelle l'élément polymère comprend en outre un plastifiant choisi dans le groupe constitué par l'eau, la glycérine, l'éthylèneglycol, le propylèneglycol, le dipropylèneglycol ou le diéthylèneglycol.

5. Composition extrudée selon la revendication 1, comprenant un élément parfumant contenant une composition parfumante.

6. Composition extrudée selon la revendication 5, dans laquelle la composition parfumante comprend au moins un ingrédient parfumant et un ou plusieurs ingrédients supplémentaires choisis dans le groupe constitué par les agents antioxydants, les agents antibactériens ou bactériostatiques, les insectifuges ou les ingrédients cosmétiques ou de soin de la peau.

7. Composition extrudée selon la revendication 1, dans laquelle la silice creuse hydrophile a une capacité d'absorption d'huile comprise entre 230ml/100g et 350ml/100g.

8. Composition extrudée selon la revendication 1, dans laquelle l'agent tensioactif est choisi dans le groupe constitué par les éthers de polyalkylèneglycol d'un alcanol en C₁ à C₁₀, les éthers de polyalkylèneglycol d'alcools gras en C₉-₂₀ synthétiques et les esters monolaurates de sorbitol condensés avec un oxyde de polyéthylène.

9. Composition extrudée selon la revendication 1, dans laquelle la teneur en parfum est comprise entre 25% et 35% du poids total de la composition.

10. Composition extrudée selon la revendication 1, comprenant en outre un colorant.

11. Composition extrudée selon l'une quelconque des revendications 1 à 10, sous une forme granulaire dans laquelle les granules ont une dimension de préférence comprise entre 0,1 mm et 10,0 mm.

12. Utilisation d'une composition extrudée selon l'une quelconque des revendications 1 à 11, en tant que moyen destiné à protéger un parfum ou une flaveur d'un milieu agressif.

13. Procédé pour parfumer une surface ou procédé pour intensifier, prolonger ou différer l'effet de diffusion de la fragrance caractéristique d'un parfum sur une surface, **caractérisé en ce que** ladite surface est traitée en présence d'une composition extrudée selon l'une quelconque des revendications 1 à 11.

14. Article de consommation ou aliment contenant une composition extrudée selon l'une quelconque des revendications 1 à 11.

15. Article de consommation selon la revendication 14, sous la forme d'un détergent en comprimés, en poudre ou en pain.

16. Aliment selon la revendication 14, sous la forme d'une gomme à mâcher.
